Europäisches Patentamt

European Patent Office

Office européen des brevets

(11) Publication number: **0 230 749**
**A1**

## (12) EUROPEAN PATENT APPLICATION

(21) Application number: **86309771.3**

(51) Int. Cl.⁴: **A61N 1/30**

(22) Date of filing: **15.12.86**

(30) Priority: **14.12.85 JP 281514/85**

(43) Date of publication of application:
**05.08.87 Bulletin 87/32**

(84) Designated Contracting States:
**DE FR GB**

(71) Applicant: **Hayashibara, Ken**
**9-8, 4-chome, Higashi-Furumatsu**
**Okayama-shi Okayama(JP)**

(72) Inventor: **Masaki, Kazumi**
**7-3, 4-chome, Fujishirodai**
**Suita-shi Osaka(JP)**

(74) Representative: **Needle, Jacqueline et al**
**PAGE, WHITE & FARRER 5 Plough Place New**
**Fetter Lane**
**London EC4A 1HY(GB)**

(54) **Therapeutic device for iontophoresing cation and anion.**

(57) A therapeutic device for iontophoresing cation and anion comprises oscillating means (A) for generating a low-frequency current in which an undirectional current is superposed on a dc current; means (S) for switching at time intervals the output polarity of said oscillating means; and electrode means (P) which contains an ionized medicament and acts as the active electrode; and further electrode means - (E) which acts as the dispersive electrode. A high efficacy which is difficult to attain by iontophoresing either cation or anion alone is possible by allowing the whole of an ionized medicament such as aminovinyl photosensitizing dye or kojic acid to penetrate into the deeper part of the skin with the therapeutic device disclosed.

FIG.6.

# THERAPEUTIC DEVICE FOR IONTOPHORESING CATION AND ANION

The present invention relates to a therapeutic device for iontophoresing cation and anion.

Endermic administration of certain medicaments such as aminovinyl photosensitizing dyes and kojic acid does not have a satisfactory efficacy because the medicaments are generally low in skin-permeability.

It has been disclosed in Japanese Laid-Open Patents Nos. 221,957/83 and 160,900/85 that the efficacy of the medicaments can be improved by iontophoresis which efficiently penetrates the medicaments into the skin.

Investigation of this phenomena has revealed that the efficacy of certain medicaments cannot be so improved by iontophoresis. We have now found that this is because in conventional devices a low-frequency current of one polarity is applied to the active electrode which has been soaked with an ionized medicament, and consequently the whole of the medicament hardly penetrates into the deeper part of the affected site.

It is an object of the present invention to provide means for efficiently iontophoresing the whole of an ionized medicament.

According to the present invention there is provided a therapeutic device for iontophoresis of both cation and anion, comprising:

means for oscillating a low-frequency current in which an undirectional current is superposed on a dc current;

means for switching at time intervals the output polarity of said oscillating means;

an electrode means which contains an ionized medicament and acts as the active electrode; and

further electrode means which acts as the dispersive electrode.

Embodiments of the present invention will hereinafter be described, by way of example, with reference to the accompanying drawings, in which:-

FIG. 1 shows the waveform of a current for iontophoresing a cation;

FIG. 2 shows the waveform of a current for iontophoresing an anion;

FIG. 3 is the molecular formula of an aminovinyl photosensitizing dye;

FIG. 4 shows the waveform of a positive superposed voltage used in iontophoresis;

FIG. 5 shows the waveform of a negative superposed voltage which is used in iontophoresis;

FIG. 6 shows a manually-operable polarity switch circuit;

FIG. 7 is a side elevation of therapeutic apparatus of the invention;

FIG. 8 shows a front elevation of another therapeutic device;

FIG. 9 shows an automatically-operable polarity switch circuit; and

FIGs. 10 and 11 show circuits for driving a polarity switch.

In all of the accompanying drawings, reference A is used to designate a low-frequency oscillator; B is a battery; C is a capacitor; E is a dispersive electrode; F is a movable iron piece; K is a power switch; L is a coil; P is a active electrode; R is a resistor; S is a polarity switch; SCR is a thyristor; and Y is a yoke.

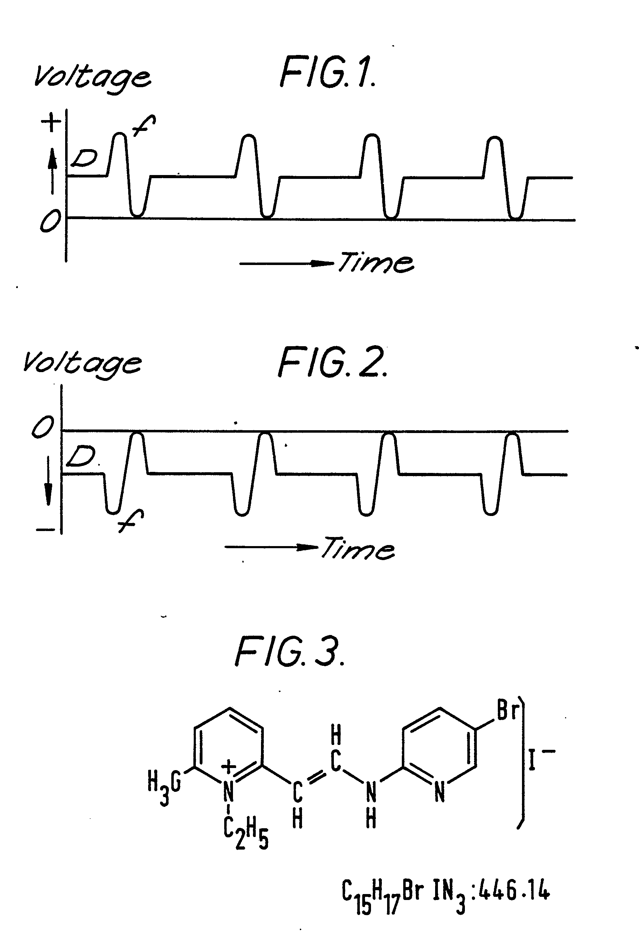

FIG. 1 illustrates a current for iontophoresing a cation in which a undirectional voltage f is superposed on a positive dc voltage D.

FIG. 2 shows a current for iontophoresing an anion having undirectional voltage f superposed on a negative dc voltage D.

The cation and iodide of an aminovinyl photosensitizing dye having the molecular formula shown in FIG. 3 can be iontophoresed respectively by the illustrated positive and negative superposed voltages. Successive application of these superposed voltages penetrates the whole molecule into the deeper part of the skin.

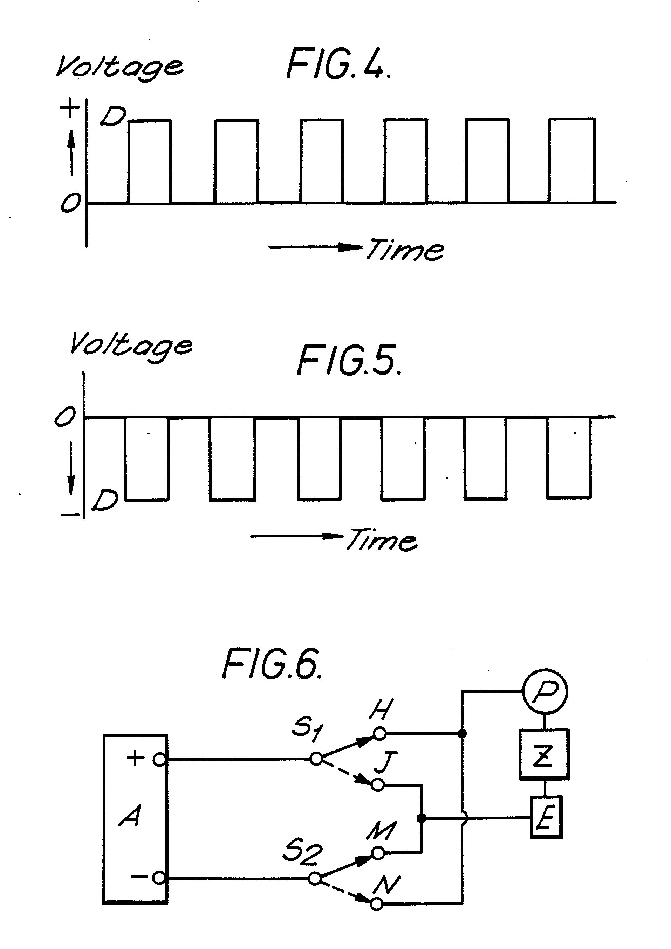

FIGs. 4 and 5 illustrate waveforms of a current for iontophoresis of the invention in which the dc voltage D is continually conducted. The positive voltage having the waveform as shown in FIG. 4 is used for iontophoresis of a cation, whilst the negative voltage having the waveform as shown in FIG. 5 is for iontophoresis of an anion.

FIG. 6 illustrates a manually-operable polarity switch circuit. When switches $S_1$ and $S_2$ make conductive contact respectively with contact H and M, a current flows from the positive output of a low-frequency oscillator A to its negative output by way of the contact H, an active electrode P, a load Z, a dispersive electrode E and the contact M.

When the positive output of the low-frequency oscillator A is connected to the dispersive electrode E by moving the switches $S_1$ and $S_2$ respectively to contact J and N as shown by broken lines, a current of reverse polarity flows from the negative output of the low-frequency oscillator A to its positive output successively by way of the load Z, the active electrode P and the contact M.

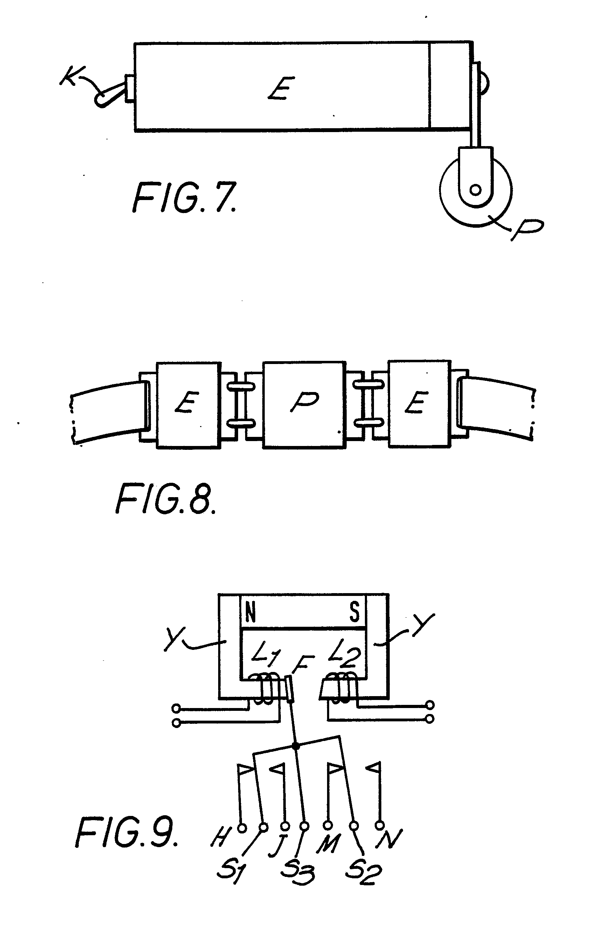

FIG. 7 shows an exterior view of a therapeutic apparatus of the invention, in which the low-frequency oscillator A and a battery are placed in a cylindrical metal body which acts as the dispersive electrode. A power switch K is attached to one end of the body, and an active electrode P is of a moisture-retentive material, such as sponge, which soaks up a medicament, is attached to the other end of the body.

FIG. 8 shows an exterior view of another therapeutic device of the invention, in which the active electrode P and the dispersive electrode E, both comprising a plate electrode and moisture-retentive material such as sponge, are jointed, and a rubber band is attached to dispersive electrode E such that the device can be positioned around the head.

FIG. 9 shows an automatically-operable polarity switch circuit having coils $L_1$ and $L_2$ wound around a yoke Y which is provided at the opposite ends of a magnet NS. In this arrangement, energization of the coil $L_1$ causes the iron piece F to move from the pole N to the pole S, and switches $S_1$ and $S_2$ are thereby switched. When the coil $L_2$ is energized, the iron piece F is immediately attracted from the pole N to the pole S.

The switch circuit of FIG. 9 can be used advantageously in a therapeutic device incorporating a battery because the circuit is switchable by a flash current and requires no holding current.

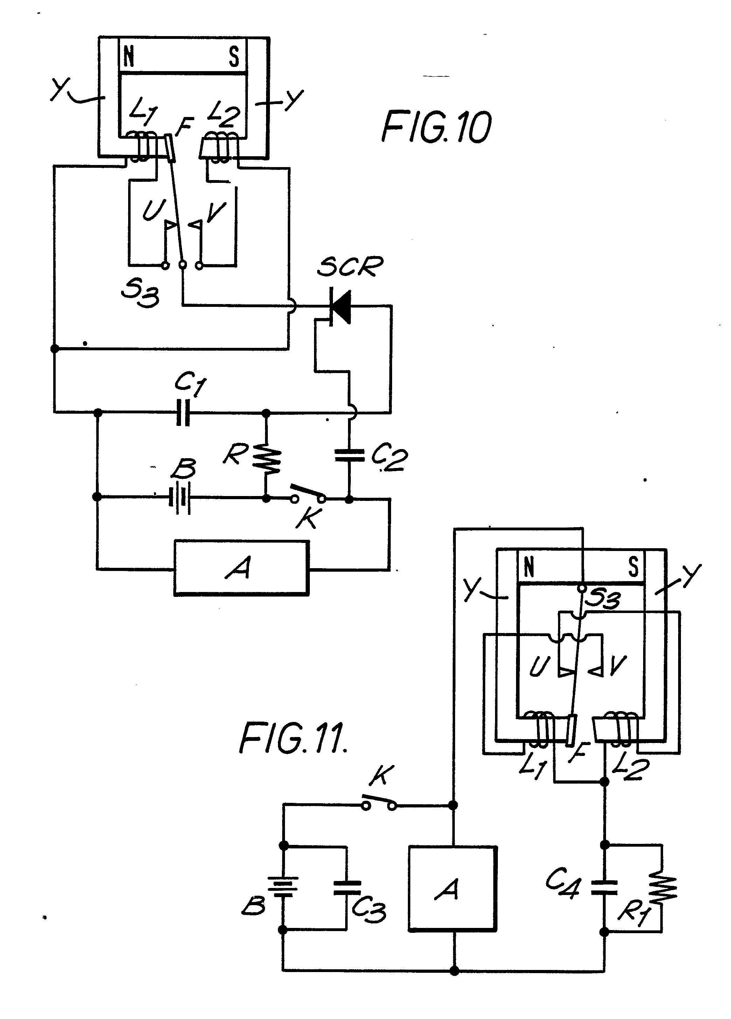

FIG. 10 illustrates a drive circuit for a polarity switch, in which a capacitor $C_1$ is charged by battery B by way of a resistor R and the voltage across the capacitor $C_1$ energizes the anode of a thyristor SCR. In this arrangement, when the power switch K is turned on and low-frequency oscillator A is energized, a pulsed voltage is immediately applied by a capacitor $C_2$ to the gate of thyristor SCR to trigger the thyristor. Conduction of the thyristor SCR energizes the coil $L_1$ to cancel the magnetic force of pole N, therefore movable iron piece F returns to the centre and is immediately attracted to pole S, while switch $S_3$ is switched to contact V. The next conduction of thyristor SCR energizes in turn coil $L_2$ to cancel the magnetic force of pole S, and movable iron piece F is attracted, inversely, to pole N.

In this way, the polarity of the voltage between active electrode P and dispersive electrode E is switched whenever the off/on operation of power switch K reverses the connection of switch $S_3$.

FIG. 11 illustrates another drive circuit for a polarity switch. In the circuit when the power switch K is closed, a current is supplied to low-frequency oscillator A and, by way of a contact U, to coil $L_2$ to cancel the magnetic force of pole S. Thus, movable iron piece F is attracted to pole N.

In this drive circuit, a capacitor $C_3$ connected in parallel with the battery B is used to decrease the internal resistance of battery B as well as to supply a high flash current. The capacitor $C_3$ is charged by way of the switches K and $S_3$, and either coil $L_1$ or $L_2$, and the change current energizes either coil $L_1$ or $L_2$ to quickly cancel the magnetic force of pole N or S to return iron piece F to the centre.

Resistor $R_1$, connected in parallel with capacitor $C_4$, is used to discharge the capacitor $C_4$. When capacitor $C_4$ and resistor $R_1$ are, for example, 100 microfarads and 100 kiloohms respectively, the time constant is 1,000 seconds. Thus, capacitor $C_4$ is discharged for up to 1,000 seconds. By adequately setting the time constant, the drive circuit is not operated when off/on operation of power switch K is carried out during the relatively short time interval, thus the output polarity of the oscillator A can be reversed by one-or two-times switchings per day.

As described above, since the whole of an ionized medicament can be efficiently iontophoresed into the deeper part of an affected site by energizing the output of a low-frequency oscillator to a pair of active-and dispersive-electrodes while switching the polarity of the output at prescribed time intervals, a high effecacy which is hardly attainable by iontophoresing either cation or anion is possible by using a therapeutic device of the invention.

Furthermore, since according to the invention the output polarity of a low-frequency oscillator is automatically switched, the hole of an ionized medicament can be easily iontophoresed without switching the output polarity with a complicated switching operation.

## Claims

1. A theraprutic device for iontophoresis of both cation and anion, comprising:
means (A) for oscillating a low-frequency current in which an unidirectional current is superposed on a dc current;
means (S) for switching at time intervals the output polarity of said oscillating means;
an electrode means (P) which contains an ionized medicament and acts as the active electrode; and
further electrode means (E) which acts as the dispersive electrode.

2. A device as claimed in Claim 1, wherein said switching means (S) is a manually-operable switch $(S_1, S_2)$.

3. A device as claimed in Claim 1, wherein said switching means (S) comprises an automatically-operable switch $(S_3)$.

4. A device as claimed in any of Claims 1 to 3, wherein said medicament is a member selected from the group consisting of aminovinyl photosensitizing dye and kojic acid.

## FIG.1.

## FIG.2.

## FIG.3.

$C_{15}H_{17}Br\ IN_3 : 446.14$

Voltage

# FIG.4.

+ D

O

→ Time

Voltage

# FIG.5.

O

D

→ Time

# FIG.6.

A

+

−

S₁

H

J

S₂

M

N

P

Z

E

FIG.7.

FIG.8.

FIG.9.

FIG.10

FIG.11.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | FR-A-2 539 622  (HAYASHIBARA) * Page 3, line 22 - page 4, line 26 * | 1 | A 61 N    1/30 |
| A | | 4 | |
| Y | DE-A-3 127 231  (COSMODERM) * Page 5, lines 4-16; page 8, line 6 - page 9, line 3 * | 1 | |
| A | | 2 | |
| A | FR-A-2 439 024  (TAPPER) * Page 2, lines 13-33; page 6, lines 9-38 * | 1,3 | |
| A | FR-A-2 307 554  (LEGUELTE) * Page 2, line 27 - page 3, line 5 * | 1,2 | TECHNICAL FIELDS SEARCHED (Int. Cl.4) A 61 N |

The present search report has been drawn up for all claims

| Place of search | Date of completion of the search | Examiner |
|---|---|---|
| THE HAGUE | 26-03-1987 | LEMERCIER D.L.L. |